# EUROPÄISCHE PATENTSCHRIFT

(11) **EP 0 910 317 B1**
(45) Veröffentlichungstag und Bekanntmachung des Hinweises auf die Patenterteilung: **11.12.2002**
(21) Anmeldenummer: 97929197.8
(22) Anmeldetag: 17.06.1997
(51) Int. Cl.: A61F 2/46, A61B 17/16, B25D 9/14, G10K 9/10

(54) **CHIRURGISCHES INSTRUMENT ZUM MECHANISCHEN ENTFERNEN VON KNOCHENZEMENT**
SURGICAL INSTRUMENT FOR MECHANICAL REMOVAL OF BONE CEMENT
INSTRUMENT CHIRURGICAL POUR L'ENLEVEMENT MECANIQUE DE CIMENT OSSEUX

(30) Priorität: 19.06.1996 DE 19624446
(43) Veröffentlichungstag der Anmeldung: 28.04.1999
(73) Patentinhaber: Ferton Holding, 2800 Delemont (CH)
(72) Erfinder: SCHMIDT, Joachim, D-51427 Bergisch Gladbach (DE); MERKLE, Wolfgang, D-52441 Linnich (DE); MENNE, Andreas, D-88709 Meersburg (DE); SIMON, Bernard, F-39220 La Cure (FR); KLOPFENSTEIN, Denis, CH-1110 Morges (CH)
(74) Vertreter: Dallmeyer, Georg, Dipl.-Ing.
(86) Internationale Anmeldenummer: EP9703143
(87) Internationale Veröffentlichungsnummer: WO97048353

(56) Entgegenhaltungen:
- EP-A- 0 144 005
- EP-A- 0 317 507
- WO-A-91/11966
- WO-A-95/22934
- DE-A- 4 416 377
- US-A- 5 027 792
- US-A- 5 057 112
- US-A- 5 221 282
- US-A- 5 300 021

## Beschreibung

Die Erfindung betrifft ein chirurgisches Instrument zum mechanischen Entfernen von Knochenzement nach dem Oberbegriff des Anspruchs 1.

Derartige chirurgische Instrumente werden bei orthophädischen Operationen benötigt, um z.B. nach dem Entfernen einer Hüftgelenkprothese den verbliebenen Knochenzement, z.B. aus PMMA zu entfernen, und einen einwandfreien Sitz der neu einzusetzenden Hüftgelenkprothese zu ermöglichen. Zum Entfernen des Zementes werden beispielsweise Meißelwerkzeuge mit Hilfe eines Hammers in den Zement hineingetrieben, um diesen von dem Knochen in der Knochenmarkhöhle abzuspalten.

Die Entfernung von Knochenzement ist vorzugsweise in der orthopädischen Chirurgie beim Wechsel oder Ausbau von gelockerten oder infizierten Endoprothesen notwendig. Andere Anwendungen sind zusätzlich möglich.

Die Zahl der Prothesenwechsel nimmt insbesondere in der Hüftendoprothetik stetig zu. Die Entfernung des Knochenzementes bei zementfixierten Prothesen ist mühsam und zeitaufwendig. Bisher wird die Zemententfernung in der Regel mit verschieden geformten Meißeln durchgeführt, deren sichere Anwendung z.B. in der schlecht einsehbaren Tiefe der Markhöhle problematisch ist. Es können neben dem hohen Zeitaufwand Knochenschädigungen resultieren, die die Neuimplantation einer Prothese unmöglich machen oder aber zu einem übergroßen Knochenverlust führen.

Es sind auch pneumatische Hämmer bekannt (EP 0 144 005, WO95/22934), bei denen ein Kolbenelement pneumatisch in einem Zylinder hin- und herbewegt wird, wobei das Kolbenelement an dem distalen Ende des Zylinders einen Schlag auf ein in dem Gehäuse axial gelagertes Meißelwerkzeug ausübt. Bei einem solchen System wird das Meißelwerkzeug auf Geschwindigkeiten von knapp über 3 m/s beschleunigt, wobei der Hub des Meißelwerkzeuges ca. 8 mm beträgt. Aufgrund des großen Hubes besteht die Gefahr, daß das Meißelwerkzeug unbeabsichtigt in die Knochensubstanz eindringt.

Mit einem solchen chirurgischen Instrument läßt sich demzufolge lediglich der bisher manuell ausgeführte Hammerschlag imitieren. Dabei hat sich allerdings herausgestellt, daß die Effizienz eines solchen pneumatisch betätigten Meißels für die Anforderungen bei der Knochenzemententfernung aus der Markhöhle nicht ausreichend ist, und daß die Gefahr einer Knochenschädigung besteht.

Es sind ferner chirurgische Instrumente bekannt, mit denen mit Hilfe von Ultraschallschwingungen thermoplastischer Knochenzement aufgeschmolzen und entfernt werden kann (US 5,221,282). Bei einem derartigen Instrument wird der Knochenzement ab ca. 100 °C duktil und kann dann entfernt werden. Dabei entstehen an der Werkzeugspitze Temperaturen von bis zu 200 °C, die eine Knochenschädigung hervorrufen können. Die bei der Knochenzemententfernung entstehende Rauchentwicklung erzeugt freiwerdende Monomere, die toxisch wirken.

Aus der EP 0 317 507 ist eine Vorrichtung bekannt, mit der Ultraschallstoßwellen übertragen werden können, wobei in einem länglichen Gehäuse ein Zylinder angeordnet ist, in dem ein aus einem Projektil bestehendes Kolbenelement mit Hilfe einer pneumatischen Antriebseinrichtung hinund herbewegbar ist, wobei das Kolbenelement an dem distalen Ende des Zylinders einen Schlag auf ein in dem Gehäuse axial gelagertes, aus einer metallischen Stoßwellenübertragungssonde bestehendes Stoßwerkzeug ausübt.

Der Erfindung liegt die Aufgabe zugrunde, ein chirurgisches Instrument zum Entfernen von Knochenzement der eingangs genannten Art anzugeben, mit dem die Entfernung von Knochenzement mit erheblich erhöhter Effektivität erfolgen kann.

Die Erfindung sieht in vorteilhafter Weise vor, daß das Kolbenelement aus einem Projektil besteht, das auf eine hohe Endgeschwindigkeit beschleunigbar ist und eine Stoßwelle in das aus einer Stoßwellenübertragungssonde bestehende Meißelwerkzeug induziert, dessen Sondenspitze die Stoßwelle auf den Knochenzement überträgt.

Bei der ballistischen Energiegewinnung sind dabei unerwünschte Hitzeentwicklungen nicht zu erwarten und die Zemententfernung ist gut steuerbar. Der Zeitaufwand zur Zemententfernung, vorzugsweise am Beispiel des Prothesen-Wechsels, wird deutlich reduziert. Eine Zemententfernung ist ohne wesentliche Schädigung des Knochens möglich.

Die Stoßwellenübertragung erfolgt demzufolge nicht durch einen Hub des gesamten Meißelwerkzeugs, sondern im wesentlichen aufgrund der durch die Stoßwelle induzierten Längenänderung der Stoßwellenübertragungssonde an der Sondenspitze. Auf diese Weise lassen sich an der Sondenspitze hohe Beschleunigungen und Geschwindigkeiten erzeugen, durch die die übertragbare Stoßenergie um ein Vielfaches höher ist als bei herkömmlichen pneumatisch betätigen Meißeln, da die Geschwindigkeit quadratisch in die Gesetzmäßigkeiten der Stoßenergieübertragung eingeht.

Die Endgeschwindkeit des auf die Stoßwellenübertragungssonde auftreffenden Projektils beträgt 5 bis 20 m/s, vorzugsweise 10 bis 15 m/s. Bei einer derartigen Auftreffgeschwindigkeit des Projektils läßt sich auch eine entsprechende Maximalgeschwindigkeit an der Sondenspitze bis zu 20 m/s feststellen.

Die Bewegungsamplitude der Sondenspitze beträgt weniger als 1,5 mm, vorzugsweise weniger als 1 mm. Die Längenänderung ist teilweise auf die elastische Verformung der Stoßwellenübertragungssonde zurückzuführen und beträgt weniger als 1,5 mm, üblicherweise zwischen 0,5 und 1 mm.

Die von dem Projektil eingebrachte Stoßwellenenergie liegt im Bereich bis zwischen 0,3 J und 2 J, vorzugsweise zwischen 0,5 J und 1,0 J.

Das Verhältnis der Projektilmasse zu Sondenmasse beträgt 1:2 bis 1:10.

Die Stoßwellen werden mit einer Schlagfrequenz des Projektils von 6 bis 20 Hz, vorzugsweise 8 bis 10 Hz, erzeugt.

Der Durchmesser der Stoßwellenübertragungssonde liegt im Bereich zwischen ca. 1 und 6 mm, vorzugsweise zwischen 2 und 4 mm.

Die Stoßwellenübertragungssonde ist axial in dem Gehäuse geführt und ein in Axialrichtung wirkendes Feder-Dämpfungselement ist zwischen Stoßwellenübertragungssonde und dem Gehäuse angeordnet. Auf diese Weise wird eine Entkopplung der Stoßwellenübertragungssonde von dem Gehäuse in Axialrichtung realisiert.

Es kann auch vorgesehen sein, daß das Projektil den Stoßimpuls auf ein Zwischenelement überträgt, das bündig an der Stoßwellenübertragungssonde anliegt.

Die Stoßwellenübertragungssonde kann hohl ausgebildet sein und das zwischenelement kann aus einer zum proximalen Ende hin geschlossenen Hülse mit mindestens einer radialen Austrittsöffnung bestehen. Mit einem derartigen Zwischenelement kann in Verbindung mit einer hohlen Stoßwellenübertragungssonde eine Absaugeinrichtung angeschlossen werden, mit der der abgelöste Knochenzement abgesaugt werden kann.

Der Beschleunigungsweg des Projektils beträgt vorzugsweise ca. 100 bis 200 mm. Damit beträgt der Beschleunigungsweg ein Vielfaches des Durchmessers des Projektils.

Die Stoßwellenübertragungssonde kann flexibel sein, so daß die Stoßwelle auch nicht geradlinig übertragen werden kann.

Desweiteren kann an dem proximalen Ende des Zylinders ein Magnethalter für das Projektil angeordnet sein. Der Magnethalter hält das Projektil in der proximalen Endlage bis es erneut gegen die Stoßwellenübertragungssonde beschleunigt wird.

Vorzugsweise wird das Projektil von pneumatischen Antriebsmitteln beschleunigt.

Die Stoßwellenübertragungssonde kann in einem Arbeitskanal eines Endoskopes geführt sein. Durch den optisch kontrollierten lokalen Einsatz der Sondenspitze auf dem Zement auch im sonst nicht einsehbaren Bereich in der Tiefe der Markhöhle wird einerseits die Effektivität der Zemententfernung erhöht, andererseits die Gefahr von unerwünschten Knochen- und Weichteilschädigungen reduziert. Zusätzliche operative Maßnahmen, wie z.B. die Anlage von Knochenfenstern, sind überflüssig.

Dabei kann auch vorgesehen sein, daß das Endoskop eine Linsenreinigungseinrichtung an dem distalen Ende aufweist. Die optische Linse des Endoskopes wird zwecks Reinigung an dem distalen Ende des Endoskopes vorzugsweise mit CO₂ gespült.

Im folgenden wird unter Bezugnahme auf die Zeichnungen ein Ausführungsbeispiel der Erfindung näher erläutert.

Es zeigen:
- Fig. 1: eine schematische Darstellung des chirurgischen Instrumentes im Querschnitt,
- Fig. 2: den Einsatz des chirurgischen Instrumentes in Verbindung mit einem Endoskop, und

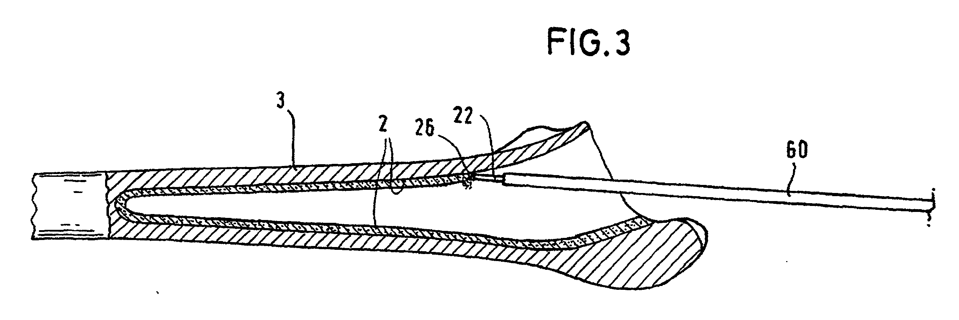

- Fig. 3: die in die Knochenzementhöhle eingeführte Endoskopsonde.

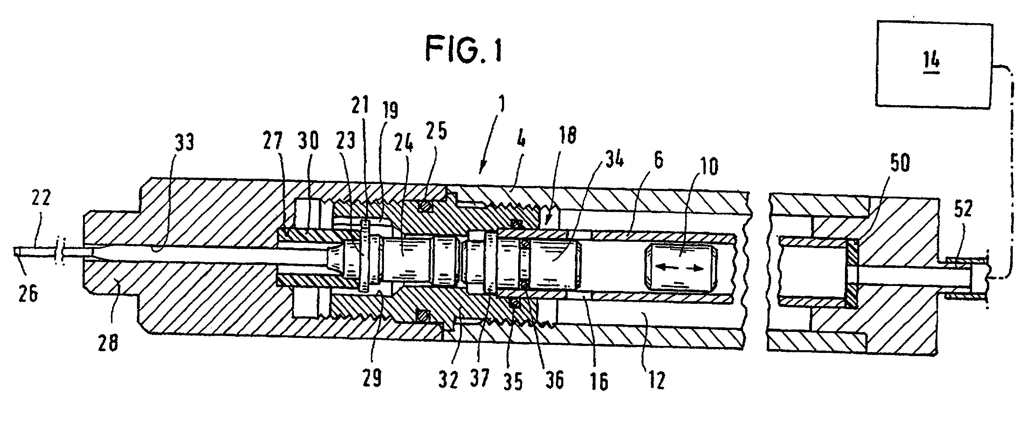

Das in Fig. 1 zeigte Handstück 1 besteht aus einem Gehäuse 4, das einen pneumatischen Zylinder 6 aufnimmt, in dem ein Projektil 10 mit Hilfe pneumatischer Antriebsmittel 14 in Verbindung mit einer Staudruckkammer 12, die den Zylinder 6 koaxial ringförmig umgibt, zwischen zwei Endpositionen hin- und herbewegt wird. Alternativ ist es auch möglich, das Projektil 10 hydraulisch, mechanisch, elektromagnetisch oder durch explosive Antriebsmittel zu beschleunigen. Bei elektromagnetischer Beschleunigung des Projektils 10 kann der Beschleunigungsweg verkürzt werden, der bei einem pneumatischen Antrieb ca. 100 bis 200 mm Länge aufweist.

In der proximalen Endposition ist am proximalen Ende des Zylinders 6 ein Magnethalter 50 angeordnet, der das metallische Projektil 10 in seiner proximalen Endposition festhalten kann bis erneut ein über den Anschluß 52 aufgebrachten pneumatischer Druck das Projektil 10 in Richtung auf das distale Ende 18 des Zylinders 6 bescheunigt. Die sich in Bewegungsrichtung des Projektils 10 vor dem Projektil 10 befindliche Luft wird über mindestens eine an dem distalen Ende des Zylinders 6 angeordnete Öffnung 16 in die Staudruckkammer 12 geleitet. Durch die Beschleunigung des Projektils 10 trifft dieses mit einer hohen Endgeschwindigkeit von beispielsweise 15 m/s auf ein in dem distalen Ende 18 des Zylinders 6 angeordnetes Zwischenstück 34, das eine Stoßübertragung auf ein bündig an dem Zwischenstück 34 anliegendes Meißelwerkzeug ermöglicht. Das Meißelwerkzeug besteht aus einer metallischen Stoßwellenübertragungssonde 22, die die über das Projektil 10 und das Zwischenstück 34 induzierte Stoßwelle auf den in einer Knochenmarkhöhle zu entfernenden Knochenzement 2 überträgt. Das Zwischenstück 34 dient dazu, das Gehäuse 4 am distalen Ende 18 hermetisch abzudichten, so daß lediglich die Stoßwellenübertragungssonde 22 und ein auf das Gehäuse 4 aufschraubbares Kopfteil 28 sterilisiert werden müssen.

Der in der Staudruckkammer 12 sich aufbauende Staudruck genügt bei Wegfall des an dem pneumatischen Anschluß 52 anliegenden Druckes, um das Projektil 10 von der distalen Endlage an dem Zwischenstück 34 in die proximale Endlage zu dem Magnethalter 50 zurückzubewegen. Der pneumatische Druck an dem Anschluß 52 kann bis zu 5 bar betragen. Das Projektil 10 kann zwecks Anpassung an bestimmte Längen der Stoßwellenübertragungssonde 22 oder zum Erzeugen einer bestimmten Charakteristik der Stoßwelle hinsichtlich Länge, Masse und maximale Aufschlaggeschwindigkeit unterschiedlich gewählt werden.

Die auf die Stoßwellenübertragungssonde übertragene Stoßenergie beträgt ca. 0,3 bis 2 J, wobei ein Wert um ca. 1 J schon zu einer hohen Effektivität bei der Knochenzemententfernung führt.

Die metallische Stoßwellenübertragungssonde 22 verläuft vorzugsweise aber nicht zwingend koaxial zu dem Zylinder 6 und dem Projektil 10. Das proximale Ende 24 der Stoßwellenübertragungssonde 22 hat vorzugsweise einen Durchmesser, der dem Durchmesser des Zwischenstücks 34 und dem Durchmesser des Projektil 10 entspricht. Die Länge des Projektils 10 ist stets größer als sein Durchmesser. Damit werden bessere Führungseigenschaften in dem Zylinder 6 erreicht. Außerdem kann mit Hilfe einer unterschiedlichen Länge des Projektils 10 die Masse des Projektils in einfacher Weise variiert werden, ohne daß der Durchmesser des Zwischenstücks 34 oder des proximalen Endes 24 der Stoßwellenübertragungssonde 22 verändert werden müssen. Das Verhältnis Länge zu Durchmesser des Projektils 10 beträgt ca. 2,5:1 bis 5:1.

Das proximale Ende 24 der Stoßwellenübertragungssonde 22 ist axial in einer Führungsöffnung 29 eines Verbindungselementes 32 gelagert, das zugleich das Zwischenstück 34 und das distale Ende 18 des Zylinders 6 aufnimmt und in das Gehäuse 4 koaxial zum Zylinder 6 einschraubbar ist. Das Zwischenstück 34 ist beweglich in dem distalen Ende 18 des Zylinders 6 angeordnet und mit einem O-Ring 36 gegen den Zylinder 6 abgedichtet. Bei Stoßbeaufschlagung des Zwischenstücks 34 durch das Projektil 10 kann sich das Zwischenstück in distaler Richtung entsprechend der Verformung des Feder/Dämpfungselementes 30 vorwärts bewegen, wobei das Zwischenstück 34 durch die Rückstellkraft des Feder/Dämpfungselementes 30 in seine proximale Endposition zurückgedrückt wird, in der ein Ringbund 37 an der distalen Stirnfläche des Zylinders 6 anliegt. Das Verbindungselement 32 ist mit einem O-Ring 35 gegen die äußere Mantelfläche des Zylinders 6 abgedichtet. Beispielsweise durch Abflachung des proximalen Endes 24 oder eines Ringbundes 23 an dem proximalen Ende 24 und einer dieser Abflachung angepaßten Führungsöffnung 29 in dem Verbindungselement 32 kann eine Verdrehsicherung für die Stoßwellenübertragungssonde 22 vorgesehen werden. In Fig. 1 ist an dem Ringbund 23 ein Vorsprung 21 vorgesehen, der als Verdrehsicherung in eine entsprechende Nut 19 des Verbindungselementes 32 eingreift.

Auf der distalen Seite des Verbindungselementes 32 ist bündig mit dem Gehäuse 4 das Kopfteil 28 aufgeschraubt, das mit einem auf dem Verbindungselement 32 sitzenden O-Ring 25 abgedichtet wird. Das Verbindungselement 32 führt den Schaft der Stoßwellenübertragungssonde 22 in einer Bohrung 33 axial und nimmt ein hülsenförmiges, z.B. aus Silikon bestehendes Feder/Dämpfungselement 30 auf. Das Feder/Dämpfungselement 30 wirkt in Axialrichtung und entkoppelt die Stoßwellenübertragungssonde 22 in Axialrichtung von dem Kopfteil 28 bzw. dem Gehäuse 4. Hierzu ist an dem proximalen Ende 24 der Stoßwellenübertragungssonde 22 ein Ringbund 23 angeformt, gegen den sich das Feder/Dämpfungselement 30 abstützen kann. Das Feder/Dämpfungselement 30 sitzt in einer zylindrischen Aussparung 27 des Kopfteils 28. In distaler Richtung hinter dem Ringbund 23 verringert sich der Durchmesser der Stoßwellenübertragungssonde 22 auf einen Schaftdurchmesser von ca. 2 bis 4 mm, z.B. 3,2 mm. Das Verhältnis zwischen Projektildurchmesser und Schaftdurchmesser beträgt ca. 2:1 bis 3:1. Bei einem solchen Verhältnis läßt sich der Bewegungshub an der Sondenspitze 26 auf einen geringen Wert begrenzen.

Die Sondenspitze 26 ist in Form eines Meißelwerkzeugs gestaltet. Die Stoßwellenübertragungssonde 22 kann auch als hohles Werkzeug ausgebildet sein, um ein Absaugen des gelösten Knochenzementes 2 zu ermöglichen.

In diesem Fall kann auch das Zwischenstück 34 als in distaler Richtung offene Hülse mit einer radialen Auslaßöffnung gestaltet sein, an die ein Absauganschluß angeschlossen werden kann.

Die Stoßwellen werden mit einer Schlagfrequenz zwischen 6 und 20 Hz erzeugt, wobei zur Knochenzemententfernung eine Schlagfrequenz von 10 Hz besonders geeignet erscheint. Die Sondenspitze 26 führt einen Bewegungshub aus, der maximal ca. 1,5 mm und vorzugsweise weniger als 1 mm beträgt. Ein Teil dieser Bewegung der Sondenspitze 26, ca. 0,3 mm ist auf eine Längung der Stoßwellenübertragungssonde 22 aufgrund der Stoßwelle zurückzuführen, während der restliche Teil des Bewegungshubes auf eine Deformation des Feder/-Dämpfungselementes 30 zurückzuführen ist. Der auf die Stoßwelle zurückzuführende Anteil beträgt dabei ca. 30 % des gesamten Bewegungshubes, der nach ca. 1 ms seinen Maximalwert erreicht. Die an den Enden der Stoßwellenübertragungssonde 22 reflektierende Stoßwelle erzeugt dem Bewegungshub der Sondenspitze 26 überlagerte Schwingungen mit steilen Anstiegsflanken, die steiler sind als die Anstiegsflanken des Bewegungshubes und damit zu einer erheblichen Erhöhung der Sondenspitzengeschwindigkeit beitragen. Auf diese Weise kann eine im Vergleich zu herkömmlichen pneumatischen Meißeln um einen Faktor von ca. 30 bis 50 höhere Stoßenergie auf den Knochenzement 2 übertragen werden.

Das Verhältnis von Projektilmasse und Sondenmasse beträgt ca. 1:2 bis 1:10, vorzugsweise ca. 1:4 bis 1:6. Die Masse des Projektils 10 ist demzufolge kleiner als die Masse der Stoßwellenübertragungssonde 22.

Die Länge des Projektils 10 bestimmt die Länge der Stoßwelle. Das Verhältnis der Projektillänge zur Sondenlänge beträgt ca. 1:10 bis 1:30, vorzugsweise ca. 1:15. Demzufolge ist die Länge der induzierten Stoßwelle klein im Verhältnis zu der Längenabmessung der Sonde.

Das Verhältnis des Sondenschaftdurchmessers zu der Sondenlänge liegt im Bereich zwischen ca. 1:50 und 1:200, vorzugsweise im Bereich zwischen 1:100 und 1:150. Dabei ist wie bereits erwähnt ein Sondenschaftdurchmesser von ca. 2 bis 4 mm bevorzugt. Eine derartige Sonde kann in den Arbeitskanal eines Endoskopes eingeführt werden und ermöglicht die Anwendung des Meißelwerkzeugs in endoskopischen Operationen.

Bei Verwendung der Stoßwellenübertragungssonde 22 in Verbindung mit einem Endoskop besteht die Möglichkeit, ständig oder intermittierend in einem flüssigen Medium zu arbeiten. Dies hat den Vorteil, daß ggf. eine Linsenreinigungseinrichtung entfallen kann. In diesem Fall ist es allerdings notwendig, mit einem geeigneten elastischen Stopfen die Knochenmarkhöhle an ihrer offenen Stelle abzudichten.

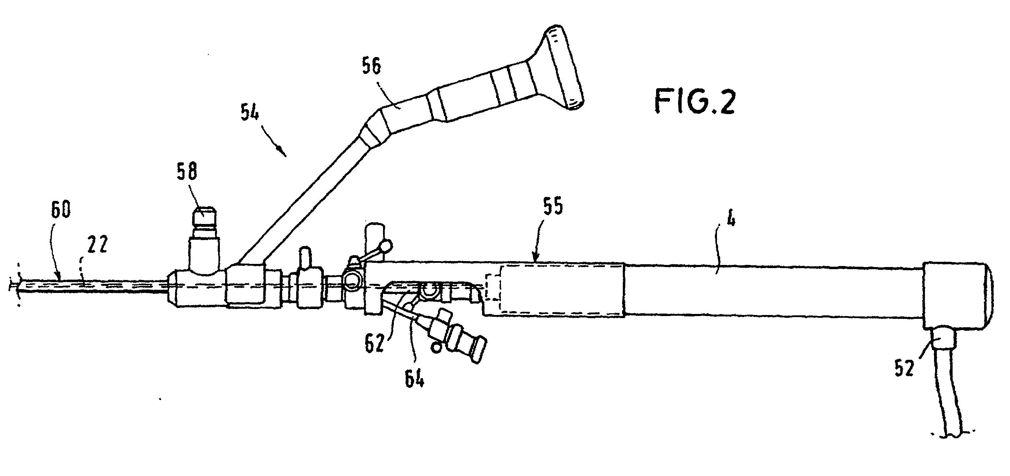

Fig. 2 zeigt den Einsatz des chirurgischen Instrumentes in einem Endoskop. Die Stoßwellenübertragungssonde 22 ist dabei in einem ersten Arbeitskanal 62 des Endoskops 54 eingesetzt. Mit Hilfe eines Okulars 56 und einer Beleuchtung durch Lichtfasern über einen Lichtfaseranschluß 58 kann das Arbeitsfeld des Meißelwerkzeugs beobachtet werden. Über einen gekrümmt in die Endoskopsonde 60 eintretenden zweiten Arbeitskanal 64 können beispielsweise Flüssigkeiten zugeführt oder abgesaugt werden. Die Ankopplung des Gehäuses 4 an das Endoskop 54 erfolgt mit Hilfe eines Adapters 55. Die Endoskopsonde 60 ermöglicht die Zemententfernung in sonst nicht einsehbaren Bereichen der Tiefe der Markhöhle. Durch die endoskopische Beobachtung des Arbeitsbereiches wird die Gefahr von unerwünschten Knochen- und Weichteilschädigungen reduziert. Zusätzliche operative Maßnahmen, wie z.B. die Anlage von Knochenfenstern sind bei endoskopischen Einsatz des chirurgischen Instrumentes nicht notwendig.

## Patentansprüche

1. Chirurgisches Instrument zum mechanischen Entfernen von Knochenzement (2), mit einem länglichen Gehäuse (4), mit einem Zylinder (6), in dem ein aus einem Projektil (10) bestehendes Kolbenelement mit Hilfe eines Antriebsmittels (14) hin- und herbewegbar ist, wobei das Kolbenelement an dem distalen Ende (18) des Zylinders (6) einen Schlag auf ein in dem Gehäuse (4) axial gelagertes, aus einer metallischen Stoßwellenübertragungssonde (22) bestehendes Meißelwerkzeug ausübt,
**dadurch gekennzeichnet,**
**daß** das Projektil (10) auf eine Endgeschwindigkeit von 5 bis 20 m/s beschleunigbar ist und eine Stoßwelle in die Stoßwellenübertragungssonde (22) induziert, deren Sondenspitze (26) die Stoßwelle bei einer Bewegungsamplitude der Sondenspitze (26) von weniger als 1,5 mm auf den Knochenzement (2) überträgt, daß die von dem Projektil (10) eingebrachte Stoßwellenenergie im Bereich zwischen 0,3 J und ca. 2 J liegt und daß das Verhältnis der Masse des Projektils (10) zu der Masse der Stoßwellenübertragungssonde (22) 1:2 bis 1:10 beträgt.

2. Instrument nach Anspruch 1, **dadurch gekennzeichnet, daß** die Endgeschwindigkeit des Projektils (10) 10 bis 15 m/s beträgt.

3. Instrument nach Anspruch 1 oder 2, **dadurch gekennzeichnet, daß** die Längenänderungsamplitude der Sondenspitze (26) weniger als 1 mm beträgt.

4. Instrument nach einem der Ansprüche 1 bis 3, **dadurch gekennzeichnet, daß** die von dem Projektil (10) eingebrachte Stoßwellenenergie im Bereich zwischen 0,5 und 1,0 J liegt.

5. Instrument nach einem der Ansprüche 1 bis 4, **dadurch gekennzeichnet, daß** die Schlagfrequenz des Projektils (10) 6 bis 20 Hz, vorzugsweise 8 bis 10 Hz, beträgt.

6. Instrument nach einem der Ansprüche 1 bis 5, **dadurch gekennzeichnet, daß** die Länge der Sonde 100 mm bis 500 mm, vorzugsweise 150 mm bis 420 mm beträgt.

7. Instrument nach einem der Ansprüche 1 bis 6, **dadurch gekennzeichnet, daß** die Stoßwellenübertragungssonde (22) in dem Gehäuse (4) in einer Führung (19,21) gegen Verdrehen gesichert ist.

8. Instrument nach einem der Ansprüche 1 bis 7, **dadurch gekennzeichnet, daß** der Durchmesser der Stoßwellenübertragungssonde (22) im Bereich zwischen 1 und 6 mm, vorzugsweise zwischen 2 und 4 mm, liegt.

9. Instrument nach einem der Ansprüche 1 bis 8, **dadurch gekennzeichnet, daß** die Stoßwellenübertragungssonde (22) axial in dem Gehäuse (4) geführt ist und ein in Axialrichtung wirkendes Feder-Dämpfungselement (30) zwischen der Stoßwellenübertragungssonde (22) und dem Gehäuse (4) angeordnet ist.

10. Instrument nach einem der Ansprüche 1 bis 9, **dadurch gekennzeichnet, daß** das Projektil (10) den Stoßimpuls auf ein Zwischenelement (34) überträgt, das bündig an der Stoßwellenübertragungssonde (22) anliegt.

11. Instrument nach Anspruch 10, **dadurch gekennzeichnet, daß** die Stoßwellenübertagungssonde (22) hohl ausgebildet ist, und daß das Zwischenelement (34) aus einer zum proximalen Ende hin geschlossenen Hülse (38) mit mindestens einer radialen Austrittsöffnung (42) besteht.

12. Instrument nach einem der Ansprüche 1 bis 11, **dadurch gekennzeichnet, daß** der Beschleunigungsweg des Projektils (10) 100 bis 200 mm beträgt.

13. Instrument nach einem der Ansprüche 1 bis 12, **dadurch gekennzeichnet, daß** an dem proximalen Ende (46) des Zylinders (6) ein Magnethalter (50) für das Projektil (10) angeordnet ist.

14. Instrument nach einem der Ansprüche 1 bis 13, **dadurch gekennzeichnet, daß** die Stoßwellenübertragungssonde (22) flexibel ist.

15. Instrument nach einem der Ansprüche 1 bis 14, **dadurch gekennzeichnet, daß** pneumatische Antriebsmittel (14) das Projektil (10) antreiben.

16. Instrument nach einem der Ansprüche 1 bis 15, **dadurch gekennzeichnet, daß** die Stoßwellenübertragungssonde (22) in einem Arbeitskanal (61) eines Endoskopes geführt ist.

17. Instrument nach Anspruch 16, **dadurch gekennzeichnet, daß** das Endoskop eine Linsenreinigungseinrichtung an dem distalen Ende aufweist.

18. Instrument nach einem der Ansprüche 1 bis 17, **dadurch gekennzeichnet, dass** das Verhältnis zwischen Projektildurchmesser und Schaftdurchmesser der Stoßwellenübertragungssonde (22) maximal 3:1 beträgt.

## Claims

1. A surgical instrument for the mechanical removal of bone cement (2), comprising a longitudinal housing (4) with a cylinder (6) in which a piston element comprising a projectile (10) can be reciprocated by a drive means (14), wherein the piston element on the distal end (18) of the cylinder (6) exerts an impact on a chisel tool supported axially in the housing (4) and comprising a metallic shock wave transmission probe (22),
**characterized in**
**that** the projectile (10) is adapted to be accelerated to a final speed of 5 to 20 m/s and to induce a shock wave into the shock wave transmission probe (22), the probe tip (26) of the shock wave transmission probe (22) transmitting the shock wave to the bone cement (2) at a movement amplitude of the probe tip (26) of less than 1.5 mm, that the shock wave energy generated by the projectile (10) is in the range between about 0.3 J and about 2 J, and that the ratio between the mass of the projectile (10) and the mass of the shock wave transmission probe (22) is about 1:2 to 1:10.

2. The instrument according to claim 1, **characterized in that** the final speed of the projectile (10) is 10 to 15 m/s.

3. The instrument according to claim 1 or 2, **characterized in that** the length-changing amplitude of the probe tip (26) is less than 1 mm.

4. The instrument according to any one of claims 1 to 3, **characterized in that** the shock wave energy generated by the projectile (10) is in the range between about 0.5 J and about 1.0 J.

5. The instrument according to any one of claims 1 to 4, **characterized in that** the impact frequency of the projectile (10) is about 6 to 20 Hz, preferably about 8 to 10 Hz.

6. The instrument according to any one of claims 1 to 5, **characterized in that** the length of the probe is about 100 mm to 500 mm, preferably about 150 mm to 420 mm.

7. The instrument according to any one of claims 1 to 6, **characterized in that**, in the housing (4), the shock wave transmission probe (22) is secured in a guide means (19,21) against torsion.

8. The instrument according to any one of claims 1 to 7, **characterized in that** the diameter of the shock wave transmission probe (22) is in the range between about 1 and 6 mm, preferably between about 2 and 4 mm.

9. The instrument according to any one of claims 1 to 8, **characterized in that** the shock wave transmission probe (22) is guided axially in the housing (4), and a damping spring element (30), acting in the axial direction, is arranged between the shock wave transmission probe (22) and the housing (4).

10. The instrument according to any one of claims 1 to 9, **characterized in that** the projectile (10) transmits the impact pulse onto an intermediate element (34) arranged in flush abutment on the shock wave transmission probe (22).

11. The instrument according to claim 10, **characterized in that** the shock wave transmission probe (22) is hollow, and that the intermediate element (34) comprises a shell (38) being closed towards the proximal end and having at least one radial outlet opening (42).

12. The instrument according to any one of claims 1 to 11, **characterized in that** the acceleration path of the projectile (10) is about 100 to 200 mm.

13. The instrument according to any one of claims 1 to 12, **characterized in that** a magnetic holder (50) for the projectile (10) is arranged on the proximal end (46) of the cylinder (6).

14. The instrument according to any one of claims 1 to 13, **characterized in that** the shock wave transmission probe (22) is flexible.

15. The instrument according to any one of claims 1 to 14, **characterized in that** pneumatic drive means (14) are provided to accelerate the projectile (10).

16. The instrument according to any one of claims 1 to 15, **characterized in that** the shock wave transmission probe (22) is guided in a working channel (61) of an endoscope.

17. The instrument according to claim 16, **characterized in that** the endoscope is provided with a lens cleansing means on the distal end.

18. The instrument according to any one of claims 1 to 17, **characterized in that** the ratio between the projectile diameter and the shaft diameter of the shock wave transmission probe (22) is 3:1 at maximum.

## Revendications

1. Instrument chirurgical pour éliminer mécaniquement du ciment osseux (2), comportant un boîtier allongé (4), un cylindre (6) dans lequel un élément de piston constitué par un projectile (10) est déplaçable suivant un mouvement alternatif à l'aide d'un moyen d'entraînement (14), l'élément de piston exerçant à l'extrémité distale (18) du cylindre (6) une percussion sur un ciseau monté axialement dans le boîtier (4) et composé par une sonde de transmission d'onde de percussion métallique (22),
**caractérisé en ce que** le projectile (10) peut être accéléré à une vitesse finale de 5 à 20 m/s et induit une onde de percussion dans la sonde de transmission d'onde de percussion (22) dont la pointe de sonde (26) transmet l'onde de percussion au ciment osseux (2) à une amplitude de mouvement de la pointe de la sonde inférieure à 1,5 mm, **en ce que** l'énergie d'onde de percussion produite par le projectile (10) est dans la plage allant de 0,3 J et 2 J environ, et **en ce que** le rapport de la masse du projectile à la masse de la sonde est compris entre 1:2 et 1:10.

2. Instrument selon la revendication 1, **caractérisé en ce que** la vitesse finale du projectile (10) est comprise entre 10 et 15 m/s.

3. Instrument selon la revendication 1 ou 2, **caractérisé en ce que** l'amplitude des variations de longueur de la pointe de sonde (26) est inférieure à 1 mm.

4. Instrument selon l'une des revendications 1 à 3, **caractérisé en ce que** l'énergie produite par le projectile (10) est dans la gamme allant de 0,5 et 1,0 J.

5. Instrument selon l'une des revendications 1 à 4, **caractérisé en ce que** la fréquence de percussion du projectile (10) est comprise entre 6 et 20 Hz, avantageusement entre 8 et 10 Hz.

6. Instrument selon l'une des revendications 1 à 5, **caractérisé en ce que** la longueur de la sonde est comprise entre 100 mm et 500 mm, avantageusement entre 150 mm et 420 mm.

7. Instrument selon l'une des revendications 1 à 6, **caractérisé en ce que** la sonde de transmission d'onde de percussion (22) est assuré contre la torsion dans un guide (19, 21) dans le boîtier (4).

8. Instrument selon l'une des revendications 1 à 7, **caractérisé en ce que** le diamètre de la sonde de transmission d'onde de percussion (22) est dans la gamme de 1 à 6 mm environ, de préférence de 2 à 4 mm.

9. Instrument selon l'une des revendications 1 à 8, **caractérisé en ce que** la sonde de transmission d'onde de percussion (22) est guidée axialement dans le boîtier (4) et un élément d'amortissement à ressort (30) agissant dans la direction axiale est disposé entre la sonde de transmission d'onde de percussion (22) et le boîtier (4).

10. Instrument selon l'une des revendications 1 à 9, **caractérisé en ce que** le projectile (10) transmet l'impulsion de percussion à un élément intermédiaire (34) qui affleure la sonde de transmission d'onde de percussion (22).

11. Instrument selon la revendication 10, **caractérisé en ce que** la sonde de transmission d'onde de percussion (22) est conformée creuse, et **en ce que** l'élément intermédiaire (34) est constitué d'une douille (38), fermée à l'extrémité proximale, dans laquelle est ménagée au moins une ouverture de sortie radiale (42).

12. Instrument selon l'une des revendications 1 à 11, **caractérisé en ce que** la distance d'accélération du projectile (10) est avantageusement comprise entre 100 et 200 mm.

13. Instrument selon l'une des revendications 1 à 12, **caractérisé en ce qu'**un support magnétique (50) destiné au projectile est disposé à l'extrémité proximale (46) du cylindre (6).

14. Instrument selon l'une des revendications 1 à 13, **caractérisé en ce que** la sonde de transmission d'onde de percussion (22) est flexible.

15. Instrument selon l'une des revendications 1 à 14, **caractérisé en ce que** des moyens d'entraînement pneumatique (14) entraînent le projectile (10).

16. Instrument selon l'une des revendications 1 à 15, **caractérisé en ce que** la sonde de transmission d'onde de percussion (22) est guidée dans un canal de travail (61) d'un endoscope.

17. Instrument selon la revendication 16, **caractérisé en ce que** l'endoscope comporte un dispositif de nettoyage de lentille à l'extrémité distale.

18. Instrument selon l'une des revendications 1 à 17, **caractérisé en ce que** le rapport entre le diamètre du projectile et le diamètre de l'arbre de la sonde de transmission d'onde de percussion (22) est au maximum de 3:1.
